# EUROPEAN PATENT SPECIFICATION

(11) **EP 4 512 447 B1**
(45) Date of publication and mention of the grant of the patent: **12.11.2025**
(21) Application number: 24710585.1
(22) Date of filing: 26.01.2024
(51) Int. Cl.: A61M 16/00

(54) **CO2 COMPENSATION APPARATUS AND SYSTEM**
CO2-GASKOMPENSATIONSVORRICHTUNG UND SYSTEM
APPAREIL ET SYSTÈME DE COMPENSATION DE CO2

(30) Priority: 03.07.2023 CN 202310804800
(43) Date of publication of application: 26.02.2025
(73) Proprietor: Guangzhou Landswick Medical Technologies Ltd., Guangzhou, Guangdong 510000 (CN)
(72) Inventor: DONG, Hui, Guangzhou, Guangdong 510000 (CN); ZHAO, Longchao, Guangzhou, Guangdong 510000 (CN); SUN, Caixin, Guangzhou, Guangdong 510000 (CN)
(74) Representative: Santarelli
(86) International application number: PCT/CN2024/074141
(87) International publication number: WO 2025/007554

(56) References cited:
- CN-A- 111 658 932
- CN-A- 113 117 206
- CN-A- 113 117 206
- CN-A- 114 733 024
- CN-A- 114 733 024
- CN-A- 114 870 179
- CN-A- 116 688 300
- CN-U- 208 710 724
- CN-U- 219 128 823
- US-A- 5 320 093
- US-A- 5 320 093
- US-A1- 2004 144 383
- US-A1- 2007 107 728
- US-A1- 2021 169 370

## Description

### Technical Field

The invention relates to the medical technology field, particularly to a CO2 compensation device and system adapted to be connected to a universal ventilator.

### Background Art

Traditional ventilators do not have CO2 compensation functionality. When used, if the patient's end-tidal carbon dioxide (EtCO2) is low and the carbon dioxide partial pressure is low, it can cause the patient to suffer from respiratory alkalosis; if the end-tidal carbon dioxide is high and the carbon dioxide partial pressure is high, it indicates CO2 retention, which can cause the patient to suffer from respiratory acidosis.

Currently, for low end-tidal carbon dioxide partial pressure (respiratory alkalosis), a bag or collection device is generally used to cover the patient's mouth and nose, allowing the patient to re-inhale the exhaled carbon dioxide. For example, an invention disclosed under the publication number "CN103536995B" reveals an oxygen mask, including a mask body and a gas storage device. A first one-way valve is set between the mask body and the gas storage device, and a first air hole and a second air hole are set between the first one-way valve and the gas storage device. The mask body and the gas storage device are connected through a pipeline structure, which includes interconnected first and second pipelines. The first pipeline has a first air hole and a third air hole on its wall, and the second pipeline has a fourth air hole. The fourth air hole is connected to the second air hole. This structure allows part of the exhaled carbon dioxide to remain in the second pipeline during exhalation. During inhalation, this part of carbon dioxide is inhaled into the first pipeline and mixed with oxygen from the storage gas device, thus avoiding discomfort caused by excessive carbon dioxide expulsion due to high oxygen concentration inhalation.

US2004144383A1 discloses gas systems and methods for enabling respiratory stability. Minimal concentrations of CO2 are mixed with pressurized air to provide a gas mix effective for stabilizing breathing of target patients or users. CO2 concentrations below about 2% and preferably between about 0.5% and 1.25% are employed. A gas modulator includes a gas mixing module, a sensor and a control processor. The gas mixing module mixes plural gases, including CO2, into a gas mix, for delivery to a substantially leak-proof patient face mask. The sensor, located substantially at the face mask, measures CO2 concentration in the face mask. The control processor, based on a signal from the sensor, controls the CO2 concentration in the gas mix.

However, the above-mentioned oxygen mask cannot precisely control the amount of carbon dioxide gas needed by the patient, nor can it precisely monitor and adjust the incoming carbon dioxide, failing to reflect the patient's end-tidal carbon dioxide situation, thus affecting the treatment effect of the ventilator.

### Summary of the Invention

The invention is set out in the appended set of claims.

Other features and advantages of this invention will be described in the following description, and, in part, will become apparent from the description, or may be learned by the practice of this invention. The objectives and other advantages of this invention may be realized and obtained through the devices particularly pointed out in the written description and claims as well as the appended drawings.

The following will further detail the technical solution of this invention through the drawings and embodiments.

### Brief Description of the Drawings

The drawings are intended to provide further understanding of the invention and constitute a part of this description, used together with the embodiments of the invention to explain the invention, without limiting the invention. In the drawings:
Figure 1 is a schematic diagram of the overall structure of a CO2 compensation device connected to a universal ventilator according to this invention;
Figure 2 is a schematic diagram of the internal structure of the compensation mixing tube in this invention;
Figure 3 is a schematic diagram of the internal structure of the respiratory gas CO2 compensator in this invention;
Figure 4 is a schematic diagram of the adjustment control system in this invention;
Figure 5 is a workflow diagram of a CO2 compensation system connected to a universal ventilator according to this invention;
Figure 6 is a schematic diagram of the internal structure of the airway in this invention;
Figure 7 is a schematic diagram of the structure of the electric heating plate in this invention;
Figure 8 is an enlarged diagram of the structure at location A in Figure 7 of this invention;
Figure 9 is a partial sectional view of the electric heating plate in this invention.

List of reference numerals: 1 - carbon dioxide gas source; 2 - ventilator; 3 - respiratory circuit; 4 - respiratory parameter detection module; 41 - flowmeter; 411- third cable; 412 - fourth cable; 42 - compensation mixing tube; 421 - connecting tube; 43 - carbon dioxide concentration sensor; 441 - intake filter; 442 - intake pressure sensor; 443 - solenoid valve; 444 - carbon dioxide regulation proportional valve; 445 - carbon dioxide flowmeter; 446 - carbon dioxide supply pressure sensor; 447 - exhaust filter; 5 - controller; 6 - patient; 7 - proximal pressure sensor; 71 - first cable; 8 - end-tidal carbon dioxide sensor; 81 - second cable; 9 - respiratory gas CO2 compensator; 10 - airway; 1001 - supporting plate; 1002 - electric heating plate; 1003 - through hole; 1004 - fixed shaft; 1005 - fixed ring; 1006 - rotating ring; 1007 - first convex ring; 1008 - second convex ring; 1009 - baffle; 1010 - drive rod; 1011 - electric telescopic rod; 1012 - strip groove; 1013 - connecting rod; 1014 - arc hole; 1015 - guide column; 1016 - limit block; 11 - gas supply circuit.

### Detailed Description of Embodiments

The following describes preferred embodiments of this invention in conjunction with the drawings. It should be understood that the preferred embodiments described herein are only for illustrating and explaining this invention and are not intended to limit this invention.

Additionally, descriptions such as "first," "second," etc., in this invention are only for descriptive purposes and are not meant to indicate or imply relative importance or the implicit indication of the number of technical features indicated. Thus, features defining "first," "second," can include one or more of such features. Furthermore, the technical solutions and technical features among various embodiments can be combined, but they must be based on the realization by those skilled in the art, and when the combination of technical solutions appears contradictory or unachievable, it should be considered that the combination of these technical solutions does not exist and is not within the scope of protection of this invention.

### Embodiment 1

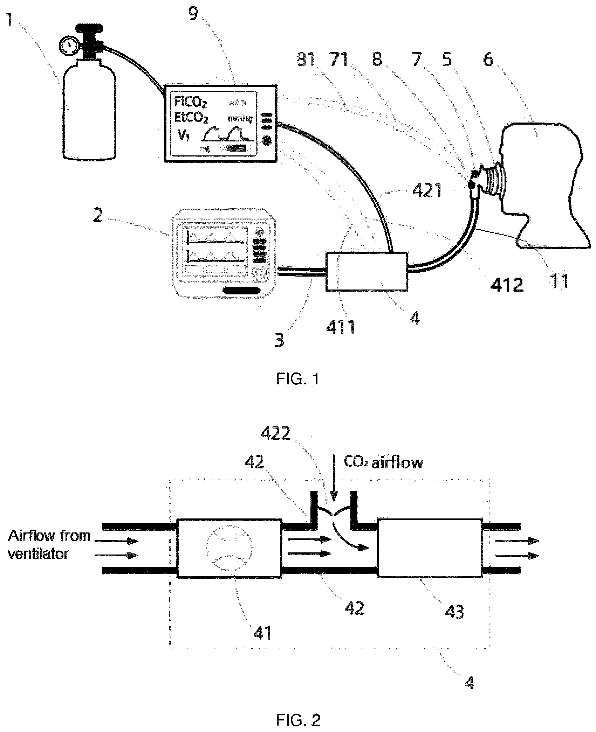

An embodiment of this invention provides a CO2 compensation device connected to a universal ventilator, as shown in Figure 1, including: the device body, which contains a respiratory gas CO2 compensator 9. Inside the respiratory gas CO2 compensator 9, an airway 10 is set up. The output end of the airway 10 is connected to a respiratory parameter detection module 4, which is connected to the respiratory terminal 5. Inside the respiratory terminal 5, a proximal pressure sensor 7 and an end-tidal carbon dioxide sensor 8 are installed. The proximal pressure sensor 7 is used to detect the gas pressure inside the respiratory terminal 5, and the end-tidal carbon dioxide sensor 8 is used to detect the end-tidal carbon dioxide value inside the respiratory terminal 5. The proximal pressure sensor 7 and the end-tidal carbon dioxide sensor 8 are electrically connected to the respiratory gas CO2 compensator 9, respectively. The respiratory gas CO2 compensator 9 delivers carbon dioxide into the respiratory terminal 5 based on the detection values of the proximal pressure sensor 7 and the end-tidal carbon dioxide sensor 8 through the respiratory parameter detection module 4.

The respiratory terminal 5 can be either a mask or a breathing tube intubation.

The working principle and beneficial effects of the above technical solution are: The device body includes a respiratory gas CO2 compensator 9, a respiratory parameter detection module 4, and a respiratory terminal 5. The respiratory terminal 5 can be either a mask or a breathing tube intubation for the patient 6 to use for breathing. The respiratory gas CO2 compensator 9 has an airway 10 set up inside, with the input end of the airway 10 able to connect to a gas source, including a carbon dioxide gas source 1 or other active gases needed by the patient 6. To prevent the patient 6 from experiencing respiratory alkalosis when using the ventilator 2, it is necessary to provide carbon dioxide to the patient 6 through the respiratory terminal 5. Taking carbon dioxide gas as an example, one end of the airway 10 is connected to the carbon dioxide gas source 1, and the other end of the airway 10 is connected to the respiratory parameter detection module 4. The output end of the respiratory parameter detection module 4 is connected to the respiratory terminal 5. At this point, starting the respiratory gas CO2 compensator 9 can allow carbon dioxide to enter the airway 10 from the input end, then flow into the respiratory parameter detection module 4 along the airway 10. The respiratory parameter detection module 4 can monitor the parameters of the carbon dioxide entering the airway 10. Then, the carbon dioxide gas flows into the respiratory terminal 5 through the output end of the respiratory parameter detection module 4, thus providing carbon dioxide to the patient 6 and avoiding respiratory alkalosis in the patient 6. The proximal pressure sensor 7 and the end-tidal carbon dioxide sensor 8 are set inside the respiratory terminal 5. The proximal pressure sensor 7 can detect the gas pressure inside the respiratory terminal 5. The end-tidal carbon dioxide sensor 8 can detect the end-tidal carbon dioxide value inside the respiratory terminal 5, thereby reflecting the patient 6's end-tidal carbon dioxide situation. The respiratory gas CO2 compensator 9 can compensate carbon dioxide gas or other active gases into the respiratory terminal 5. The respiratory parameter detection module 4 can monitor the parameters of the incoming carbon dioxide. The respiratory gas CO2 compensator 9, in coordination with the respiratory parameter detection module 4, achieves precise monitoring and adjustment of the incoming carbon dioxide, thereby accurately controlling the amount of carbon dioxide gas needed by the patient 6 and improving the treatment effect of the ventilator 2.

### Embodiment 2

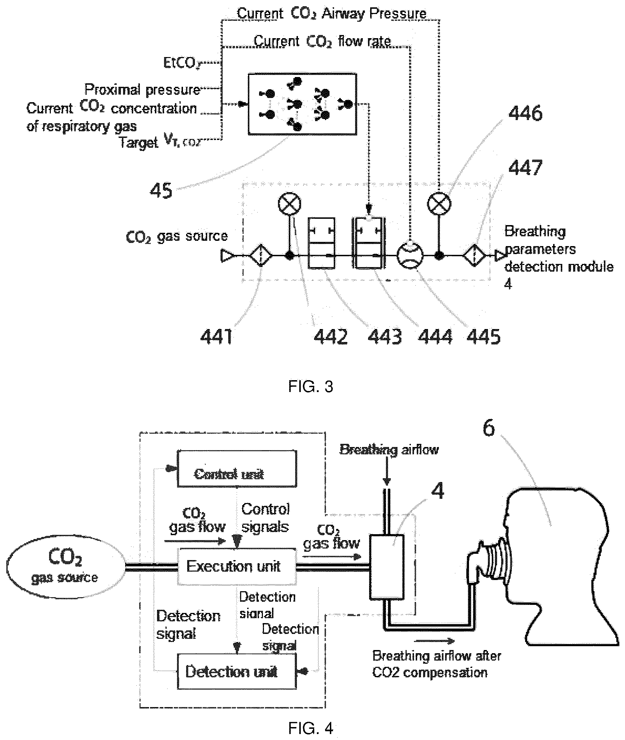

Based on Embodiment 1, as shown in Figures 1-5, the airway 10 has an intake filter 441 set near the input end and an exhaust filter 447 set near the output end.

The device body also includes an adjustment control system, which consists of a control unit, an execution unit, and a detection unit. The control unit includes a controller 45, which is set inside the respiratory gas CO2 compensator 9. The controller 45 is electrically connected to the proximal pressure sensor 7 through the first cable 71 and to the end-tidal carbon dioxide sensor 8 through the second cable 81. The execution unit includes a solenoid valve 443 and a carbon dioxide regulation proportional valve 444, both set on the airway 10. The solenoid valve 443 and the carbon dioxide regulation proportional valve 444 are sequentially set between the intake filter 441 and the exhaust filter 447 and are electrically connected to the controller 45, respectively. The detection unit includes an intake pressure sensor 442, a carbon dioxide flowmeter 445, and a carbon dioxide supply pressure sensor 446, sequentially set on the airway 10. The intake pressure sensor 442, carbon dioxide flowmeter 445, and carbon dioxide supply pressure sensor 446 are electrically connected to the controller 45, respectively. The intake pressure sensor 442 is located between the intake filter 441 and the solenoid valve 443, the carbon dioxide flowmeter 445 is located between the carbon dioxide regulation proportional valve 444 and the exhaust filter 447, and the carbon dioxide supply pressure sensor 446 is located between the carbon dioxide flowmeter 445 and the exhaust filter 447.

The respiratory parameter detection module 4 includes a compensation mixing tube 42. The compensation mixing tube 42 uses a three-way tube. The first end of the compensation mixing tube 42 is used for the airflow from the ventilator 2. The second end of the compensation mixing tube 42 is connected to the output end of the airway 10 through a connecting tube 421. A one-way valve 422 is set inside the second end of the compensation mixing tube 42. The third end of the compensation mixing tube 42 is connected to the interior of the respiratory terminal 5 through a gas supply circuit 11.

The detection unit also includes a flowmeter 41 and a carbon dioxide concentration sensor 43. The flowmeter 41 is set near the first end of the compensation mixing tube 42, and the carbon dioxide concentration sensor 43 is set near the third end of the compensation mixing tube 42. The flowmeter 41 is electrically connected to the controller 45 through the third cable 411, and the carbon dioxide concentration sensor 43 is electrically connected to the controller 45 through the fourth cable 412.

The working principle and beneficial effects of the above technical solution are: The device body also includes an adjustment control system, which consists of a control unit, an execution unit, and a detection unit. The control unit can send control signals to the execution unit. After receiving the control signals, the execution unit can allow the carbon dioxide gas flow from the carbon dioxide gas source 1 to enter the airway 10 and pass through the airway 10 to enter the compensation mixing tube 42 through its second end. The carbon dioxide entering the compensation mixing tube 42 mixes with the breathing airflow, thus achieving carbon dioxide compensation. The compensated breathing airflow then flows out from the third end into the respiratory terminal 5 for the patient 6 to breathe. The detection unit can obtain detection signals when carbon dioxide passes through the execution unit and the respiratory parameter detection module 4, and sends these detection signals to the control unit. The control unit then controls the execution unit based on the detection signals. Specifically, the control unit includes a controller 45 set inside the respiratory gas CO2 compensator 9. The execution unit includes a solenoid valve 443 and a carbon dioxide regulation proportional valve 444 set on the airway 10. The opening and closing of the solenoid valve 443 can control the connection and disconnection of the airway 10. The carbon dioxide regulation proportional valve 444 can adjust the pressure and flow of the carbon dioxide gas flow inside the airway 10. The detection unit includes an intake pressure sensor 442, a carbon dioxide flowmeter 445, a carbon dioxide supply pressure sensor 446 set on the airway 10, and a flowmeter 41 and a carbon dioxide concentration sensor 43 set inside the compensation mixing tube 42. When the respiratory terminal 5 needs carbon dioxide compensation, the controller 45 starts the solenoid valve 443 to connect the airway 10. The carbon dioxide gas flow provided by the carbon dioxide gas source 1 enters the airway 10 after passing through the intake filter 441. The intake pressure sensor 442 can detect the intake pressure of the carbon dioxide gas flow entering the airway 10. The carbon dioxide flowmeter 445 can detect the flow of the carbon dioxide gas flow inside the airway 10. The carbon dioxide supply pressure sensor 446 can detect the pressure of the carbon dioxide gas flow adjusted by the carbon dioxide regulation proportional valve 444. The adjusted carbon dioxide gas flow then passes through the exhaust filter 447 and enters the compensation mixing tube 42 through the connecting tube 421. A one-way valve 422 is set at the second end of the compensation mixing tube 42. The one-way valve 422 prevents the breathing airflow from the ventilator 2 from flowing into the connecting tube 421 through the second end. The carbon dioxide gas flow entering the compensation mixing tube 42 can mix with the breathing airflow provided by the ventilator 2, compensating for the carbon dioxide that is less in the original breathing airflow. The flowmeter 41 can detect the gas flow inside the compensation mixing tube 42. The carbon dioxide concentration sensor 43 can detect the carbon dioxide concentration of the compensated breathing airflow inside the compensation mixing tube 42 and send the detection results to the controller 45. Specifically, before use, the respiratory gas CO2 compensator 9 is set. The respiratory gas CO2 compensator 9 has a display screen that can display respiratory parameters such as VT, RR, Ti, I:E, FiO2, FiCO2, etc. VT represents tidal volume, RR represents ventilation frequency, Ti represents inhalation time, I:E represents the inhalation to exhalation ratio, FiO2 represents the fraction of oxygen concentration in the inhaled gas, and FiCO2 represents the fraction of carbon dioxide concentration in the inhaled gas. The display screen allows for a direct observation of the above parameters. The following is the working process of the device body, with the respiratory gas CO2 compensator 9 abbreviated as this device. First, set VT, RR, Ti, I:E, FiO2, or FiCO2 on this device. This device calculates the volume of carbon dioxide needed for compensation. During calculation, the volume of carbon dioxide in the exhaled gas can be obtained through the detection value of the end-tidal carbon dioxide sensor 8. Then, the volume of carbon dioxide needed for compensation can be obtained by subtracting the volume of carbon dioxide in the exhaled gas from the set volume of carbon dioxide. The set volume of carbon dioxide needs to be set according to the needs of different patients 6. Next, set the breathing mode, VT, RR, Ti, I:E, FiO2, or FiCO2, and other respiratory parameters on the ventilator 2. Check the respiratory parameters and send a status signal to this device. Start the delivery of respiratory gas. Then, this device reads the values of the flowmeter 41 and the proximal pressure sensor 7 to determine whether there is delivery of respiratory gas. If it is determined that there is no delivery of respiratory gas, repeat reading the values. If it is determined that there is delivery of respiratory gas, this device delivers the compensated carbon dioxide according to the set respiratory parameters based on the detection value of the carbon dioxide concentration sensor 43, using a carbon dioxide adjustment algorithm. The carbon dioxide adjustment algorithm requires that the sum of the compensated carbon dioxide concentration and the initial detection concentration of the carbon dioxide concentration sensor 43 be within the target carbon dioxide concentration range required in the patient's 6 respiratory airflow. The target carbon dioxide concentration range is set according to the needs of different patients 6. Then, this device reads the values of the flowmeter 41 and the proximal pressure sensor 7 to determine whether the delivery of respiratory gas has ended. If it is determined that the delivery has not ended, repeat reading the values. If it is determined that the delivery of respiratory gas has ended, this device reads the values of the flowmeter 41 and the proximal pressure sensor 7 to determine whether there is exhaled gas. If it is determined that there is no exhaled gas, repeat reading the values. If it is determined that there is exhaled gas, this device reads the end-tidal carbon dioxide value detected by the end-tidal carbon dioxide sensor 8 and corrects the volume of carbon dioxide needed for compensation based on the volume of carbon dioxide in the exhaled gas. This achieves precise control of the volume of carbon dioxide gas, as the invention automatically adjusts the volume and flow of carbon dioxide gas entering the universal ventilator 2 pipeline based on the gas source, feedback, monitoring, and sampling of the patient's 6 end-tidal carbon dioxide detection information, enhancing the automation, intelligence, and controllability of the device. Through high sensitivity CO2 monitoring and adjustment control systems, the invention provides high precision carbon dioxide gas concentration needed by the patient 6, achieving effective treatment and medical purposes, and improving treatment outcomes.

### Embodiment 3

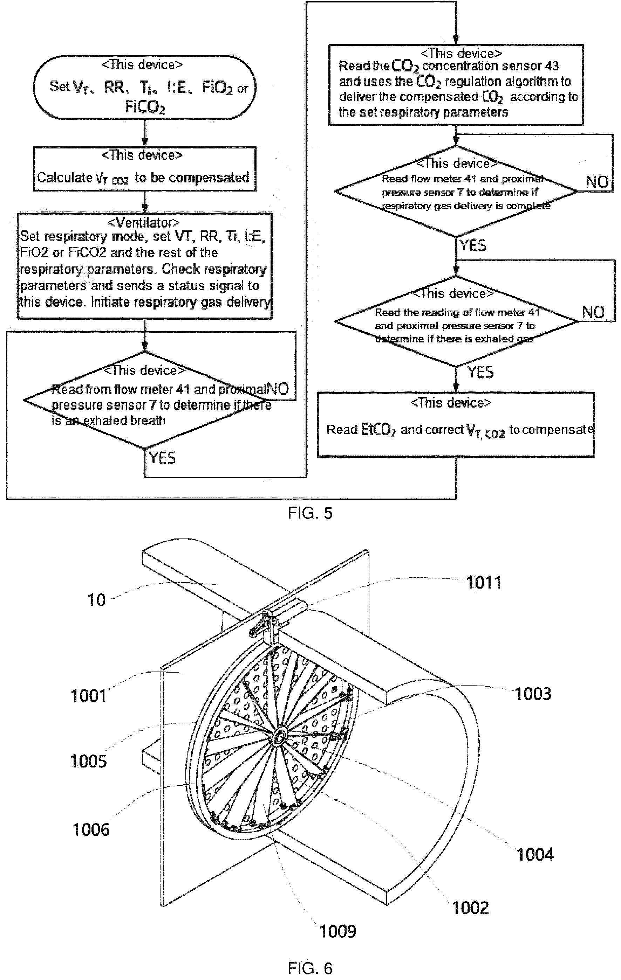

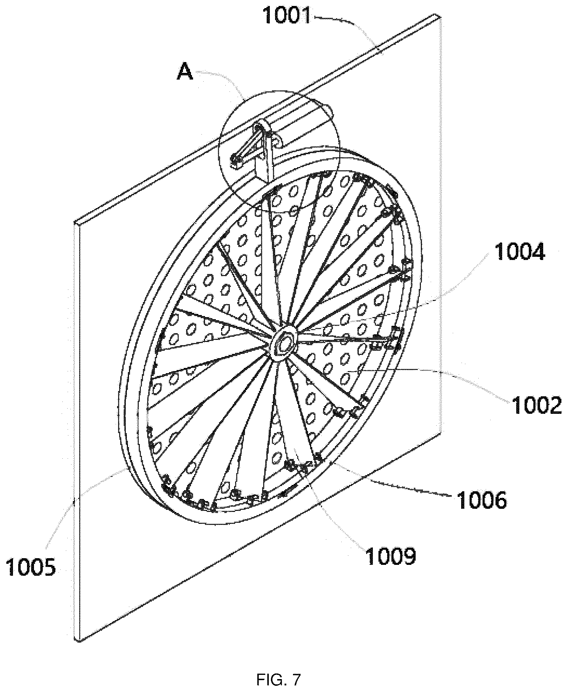

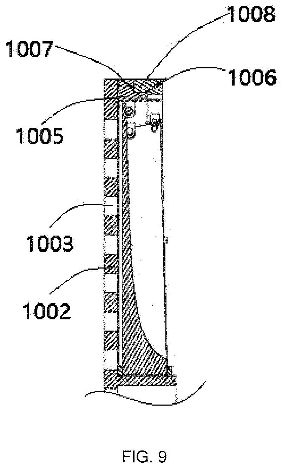

On the basis of Embodiment 2, as shown in Figures 6-9, a supporting plate 1001 is arranged outside the airway 10, and several bolt mounting holes are set on the supporting plate 1001. Inside the airway 10, an electric heating plate 1002 is arranged between the intake filter 441 and the intake pressure sensor 442. The electric heating plate 1002 is electrically connected to the controller 45 and is coaxially arranged inside the airway 10. The electric heating plate 1002 has several through holes 1003. In the center of the electric heating plate 1002, a fixed shaft 1004 is set. Near the fixed shaft 1004, a fixed ring 1005 and a rotating ring 1006 are arranged in sequence. The outer circumference of the fixed ring 1005 is fixedly connected to the inner wall of the airway 10. On the side of the fixed ring 1005 away from the electric heating plate 1002, a first convex ring 1007 is set, which is coaxially arranged with the fixed ring 1005. The outer circumference of the rotating ring 1006 is rotatably connected to the inner wall of the airway 10. On the side of the rotating ring 1006 close to the fixed ring 1005, a second convex ring 1008 is set, whose inner wall is rotatably connected to the outer wall of the first convex ring 1007. Several baffles 1009 are arranged around the outer circumference of the fixed shaft 1004. The baffles 1009 are made of flexible sheet material. Several baffles 1009 are arranged in a circular array around the center of the fixed shaft 1004. One end of the baffle 1009 is connected to the outer circle of the fixed shaft 1004, and the end of the baffle 1009 away from the fixed shaft 1004 is close to the side of the fixed ring 1005 and connected to the inner circumferential surface of the fixed ring 1005. The end of the baffle 1009 away from the fixed shaft 1004 and close to the side of the rotating ring 1006 is connected to the inner circumferential surface of the rotating ring 1006. A driving component is arranged outside the airway 10, which is used to drive the rotating ring 1006 to rotate.

The driving component includes: a drive rod 1010 and an electric telescopic rod 1011. One end of the drive rod 1010 passes through the side wall of the airway 10 and connects to the outer wall of the rotating ring 1006. A long slide slot compatible with the drive rod 1010 is set on the airway 10, and the drive rod 1010 is slidably connected to the long slide slot. The end of the drive rod 1010 away from the rotating ring 1006 is equipped with a strip groove 1012. The electric telescopic rod 1011 is arranged on the side wall of the supporting plate 1001, and its output end passes through the strip groove 1012 and is equipped with a connecting rod 1013. One end of the connecting rod 1013 is hingedly connected to the output end of the electric telescopic rod 1011, and the other end of the connecting rod 1013 is hingedly connected to the inner wall of the upper end of the strip groove 1012.

The working principle and beneficial effects of the above technical solution are as follows: Traditional ventilators 2 can adjust the temperature of the respiratory airflow, thus providing patients 6 with suitable respiratory airflow. When compensating the respiratory airflow with carbon dioxide, the temperature of the carbon dioxide airflow being too high or too low can cause significant changes in the temperature of the respiratory airflow, thereby causing damage to the airway and affecting the respiratory experience of the patient 6. Therefore, this solution arranges an electric heating plate 1002 inside the airway 10. The controller 45 can control the electric heating plate 1002 to heat up. When the carbon dioxide airflow passes through the through holes 1003 of the electric heating plate 1002, the passing carbon dioxide airflow can be heated. Setting multiple through holes 1003 can improve the heating efficiency of the carbon dioxide airflow, making the temperature of the carbon dioxide airflow entering the airway 10 consistent with the temperature of the respiratory airflow flowing out of the ventilator 2. The supporting plate 1001 is set on the outer wall of the airway 10, and the supporting plate 1001 can be fixed through bolt installation, thus fixing the airway 10 and improving the stability of the airway 10, avoiding the separation of the airway 10 from the carbon dioxide gas source 1 and affecting the compensation of the carbon dioxide airflow, thereby improving safety and reliability. When the flow rate of carbon dioxide inside the airway 10 needs to be significantly adjusted, the electric telescopic rod 1011 is activated, which then drives the connecting rod 1013 to move. The connecting rod 1013 then drives the drive rod 1010 to move, and the drive rod 1010 drives the rotating ring 1006 to rotate inside the airway 10. As the rotating ring 1006 rotates, it causes one side of the baffle 1009 to deflect, thereby adjusting the angle of the baffle 1009, changing the direction and flow rate of the passing carbon dioxide airflow, and then finely adjusting it through the carbon dioxide regulation proportional valve 444. This not only improves the adjustment precision but also increases the adjustment speed, facilitating rapid adjustments. As the rotating ring 1006 rotates, the second convex ring 1008 and the first convex ring 1007 also rotate relative to each other. The second convex ring 1008 and the first convex ring 1007 cooperate with each other, which can improve the sealing at the connection between the rotating ring 1006 and the fixed ring 1005, avoiding the leakage of carbon dioxide airflow.

### Embodiment 4

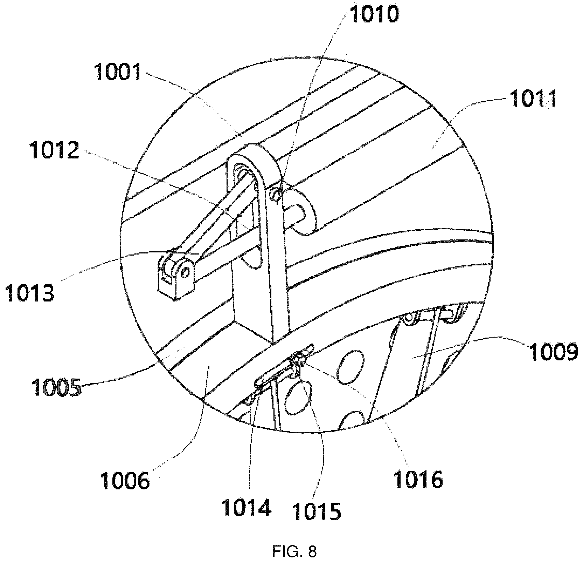

Based on Embodiment 3, as shown in Figure 8, several arc holes 1014 are opened on the rotating ring 1006, and several arc holes 1014 are arranged in a circular array around the center of the rotating ring 1006. A guide column 1015 is slidably arranged inside the arc hole 1014, one end of the guide column 1015 is fixedly connected to the side of the fixed ring 1005 close to the rotating ring 1006, and the other end of the guide column 1015 extends outside the arc hole 1014 and is equipped with a limit block 1016.

The working principle and beneficial effects of the above technical solution are as follows: The arc hole 1014 is coaxially arranged with the rotating ring 1006. When the rotating ring 1006 rotates, the arc hole 1014 can slide along the guide column 1015, improving the stability of the rotation of the rotating ring 1006. Moreover, the limit block 1016 can limit the rotating ring 1006 to prevent it from separating from the fixed ring 1005, thereby extending the service life of the device.

### Embodiment 5

Based on any one of Embodiments 1-4, as shown in Figure 1, the invention also provides a CO2 compensation system connected to a universal ventilator, including the CO2 compensation device connected to a universal ventilator mentioned above, as well as the ventilator 2 and the carbon dioxide gas source 1. The carbon dioxide gas source 1 is connected to the input end of the airway 10 of the respiratory gas CO2 compensator 9 through the intake pipe, and the ventilator 2 is connected to the compensation mixing tube 42 through the respiratory circuit 3.

The working principle and beneficial effects of the above technical solution are as follows: The device body of the invention can be connected to all existing universal ventilators 2 on the market, enhancing their functionality and increasing their applicability. The device body can provide ports for supplying carbon dioxide and other active gases to patients 6 for all ventilators 2 on the market, thus expanding the functionality of the ventilator 2, enabling it to provide the required carbon dioxide for patients 6, avoiding respiratory alkalosis in patients 6, greatly improving the therapeutic effect of the ventilator 2, and the device body can adjust the carbon dioxide supply amount and automatically regulate the flow supply according to the changes in airflow parameters such as flow and pressure in the respiratory circuit 3 of the ventilator 2, the proximal pressure, and the end-tidal carbon dioxide value, improving the intelligence and automation level of the system. Moreover, there is no need for communication between the device body and the ventilator 2, which facilitates installation without the need for modifications to the ventilator 2, enhancing its universality.

## Claims

1. A CO2 compensation device adapted to be connected to a universal ventilator (2),
wherein it includes: a device body, the device body comprises a respiratory gas CO2 compensator (9), a respiratory parameter detection module (4), and a respiratory terminal (5), an airway (10) is set inside the respiratory gas CO2 compensator (9), an output end of the airway (10) is connected to the respiratory parameter detection module (4), the respiratory parameter detection module (4) is connected to the respiratory terminal (5), a proximal pressure sensor (7) and an end-tidal carbon dioxide sensor (8) are set inside the respiratory terminal (5), the proximal pressure sensor (7) is used to detect a gas pressure inside the respiratory terminal (5), the end-tidal carbon dioxide sensor (8) is used to detect an end-tidal carbon dioxide value inside the respiratory terminal (5), the proximal pressure sensor and the end-tidal carbon dioxide sensor are electrically connected to the respiratory gas CO2 compensator (9) respectively, the respiratory gas CO2 compensator (9) delivers carbon dioxide to an inside of the respiratory terminal (5) based on detection values of the proximal pressure sensor (7) and the end-tidal carbon dioxide sensor (8) through the respiratory parameter detection module (4);
wherein an intake filter (441) is set near an input end of the airway (10), and an exhaust filter (447) is set near the output end of the airway;
wherein the device body also includes a regulation control system, the regulation control system includes a control unit, an execution unit, and a detection unit, the control unit includes a controller (45), the controller is set inside the respiratory gas CO2 compensator (9), the controller is electrically connected to the proximal pressure sensor (7) through a first cable (71), the controller is electrically connected to the end-tidal carbon dioxide sensor (8) through a second cable (81), the execution unit includes a solenoid valve (443) and a carbon dioxide regulation proportional valve (444), both the solenoid valve and the carbon dioxide regulation proportional valve are set on the airway (10), the solenoid valve and the carbon dioxide regulation proportional valve are sequentially set between the intake filter (441) and the exhaust filter (447), the solenoid valve and the carbon dioxide regulation proportional valve are electrically connected to the controller (45) respectively, the detection unit includes an intake pressure sensor (442), a carbon dioxide flowmeter (445), and a carbon dioxide supply pressure sensor (446) set in sequence on the airway (10), the intake pressure sensor (442), the carbon dioxide flowmeter (445), and the carbon dioxide supply pressure sensor (446) are electrically connected to the controller (45) respectively, the intake pressure sensor (442) is located between the intake filter (441) and the solenoid valve (443), the carbon dioxide flowmeter (445) is located between the carbon dioxide regulation proportional valve (444) and the exhaust filter (447), the carbon dioxide supply pressure sensor (446) is located between the carbon dioxide flowmeter (445) and the exhaust filter (447);
wherein the respiratory parameter detection module (4) includes a compensation mixing tube (42), the compensation mixing tube uses a three-way tube, a first end of the compensation mixing tube (42) is used for airflow from the ventilator (2), a second end of the compensation mixing tube is connected to the output end of the airway (10) through a connecting tube (421), a one-way valve (422) is set inside the second end of the compensation mixing tube (42), a third end of the compensation mixing tube (42) is connected to the inside of the respiratory terminal (5) through a gas supply circuit (11);
wherein the detection unit also includes a flowmeter (41) and a carbon dioxide concentration sensor (43), the flowmeter (41) is set near the first end of the compensation mixing tube (42), the carbon dioxide concentration sensor (43) is set near the third end of the compensation mixing tube (42), the flowmeter is electrically connected to the controller (45) through a third cable (411), the carbon dioxide concentration sensor is electrically connected to the controller (45) through a fourth cable (412).

2. The CO2 compensation device adapted to be connected to a universal ventilator (2) according to claim 1, wherein the respiratory terminal (5) is either a mask or a breathing tube intubation.

3. The CO2 compensation device adapted to be connected to a universal ventilator (2) according to claim 1, wherein an outside of the airway (10) is equipped with a supporting plate (1001), several bolt mounting holes are set on the supporting plate, an inside of the airway (10) is equipped with an electric heating plate (1002), the electric heating plate is located between the intake filter (441) and the intake pressure sensor (442), the electric heating plate is electrically connected to the controller (45), the electric heating plate is coaxially set inside the airway (10), several through holes (1003) are opened on the electric heating plate (1002), a fixed shaft (1004) is set in the center of the electric heating plate, a fixed ring (1005) and a rotating ring (1006) are sequentially set on a side of the electric heating plate (1002) near the fixed shaft (1004), an outer circumference of the fixed ring (1005) is fixedly connected to an inner wall of the airway (10), a first convex ring (1007) is set on a side of the fixed ring (1005) away from the electric heating plate (1002), the first convex ring is coaxially set with the fixed ring, an outer circumference of the rotating ring (1006) is rotatably connected to the inner wall of the airway (10), a second convex ring (1008) is set on a side of the rotating ring (1006) near the fixed ring (1005), an inner side wall of the second convex ring (1008) is rotatably connected to an outer side wall of the first convex ring (1007), several baffles (1009) are set on an outer circumference of the fixed shaft (1004), the baffles (1009) are made of flexible sheet material and are arranged in a circular array around the center of the fixed shaft, one end of each of the baffles (1009) is connected to an outer circle of the fixed shaft, an end of each of the baffles (1009) away from the fixed shaft is connected to an inner circumferential surface of the fixed ring on a side near the fixed ring, the end of each of the baffles (1009) away from the fixed shaft is connected to an inner circumferential surface of the rotating ring(1006) on a side near the rotating ring, a driving component is set outside the airway, the driving component is used to drive the rotating ring (1006) to rotate.

4. The CO2 compensation device adapted to be connected to a universal ventilator (2) according to claim 3, wherein the driving component includes: a drive rod (1010) and an electric telescopic rod (1011), one end of the drive rod penetrates a side wall of the airway (10) and is connected to an outer wall of the rotating ring (1006), a long slot compatible with the drive rod (1010) is set on the airway, the drive rod is slidably connected to the long slot, a strip groove (1012) is set at an end of the drive rod (1010) away from the rotating ring (1006), the electric telescopic rod (1011) is set on a side wall of the supporting plate (1001), an output end of the electric telescopic rod passes through the strip groove (1012) and is equipped with a connecting rod (1013), one end of the connecting rod (1013) is hingedly connected to the output end of the electric telescopic rod (1011), the other end of the connecting rod is hingedly connected to an inner wall of an upper end of the strip groove (1012).

5. The CO2 compensation device adapted to be connected to a universal ventilator (2) according to claim 3, wherein several arc holes (1014) are opened on the rotating ring (1006),
the arc holes (1014) are arranged in a circular array around the center of the rotating ring (1006), a guide column (1015) is slidably set inside each of the arc holes (1014), one end of the guide column is fixedly connected to a side of the fixed ring (1005) near the rotating ring (1006), the other end of the guide column extends outside a respective one of the arc holes (1014) and is equipped with a limit block (1016).

6. A CO2 compensation system comprising any one of the CO2 compensation devices described in claims 1-5, wherein it also includes the ventilator (2) and a carbon dioxide gas source (1), the carbon dioxide gas source (1) is connected to the input end of the airway (10) of the respiratory gas CO2 compensator (9) through an intake pipe, the ventilator (2) is connected to the compensation mixing tube (42) through a respiratory circuit (13).

## Patentansprüche

1. CO2-Kompensationsvorrichtung, die zum Anschluss an einen Universalventilator (2) ausgelegt ist, wobei
sie Folgendes enthält: einen Vorrichtungskörper, der Vorrichtungskörper umfasst einen Atemgas-CO2-Kompensator (9), ein Atemparameter-Erkennungsmodul (4) und ein Atemterminal (5), ein Atemweg (10) ist in dem Atemgas-CO2-Kompensator (9) eingerichtet, ein Ausgangsende des Atemwegs (10) ist mit dem Atemparameter-Erkennungsmodul (4) verbunden, das Atemparameter-Erkennungsmodul (4) ist mit dem Atemterminal (5) verbunden, ein proximaler Drucksensor (7) und ein endtidaler Kohlendioxidsensor (8) sind in dem Atemterminal (5) eingerichtet, der proximale Drucksensor (7) wird verwendet, um einen Gasdruck in dem Atemterminal (5) zu erfassen, der endtidale Kohlendioxidsensor (8) wird verwendet, um einen endtidalen Kohlendioxidwert in dem Atemterminal (5) zu erfassen, der proximale Drucksensor und der endtidale Kohlendioxidsensor sind jeweils elektrisch mit dem Atemgas-CO2-Kompensator (9) verbunden, der Atemgas-CO2-Kompensator (9) liefert Kohlendioxid an eine Innenseite des Atemterminals (5) basierend auf Erkennungswerten des proximalen Drucksensors (7) und des endtidalen Kohlendioxidsensors (8) durch das Atemparameter-Erkennungsmodul (4);
wobei ein Einlassfilter (441) in der Nähe eines Eingangsendes des Atemwegs (10) eingerichtet ist und ein Auslassfilter (447) in der Nähe des Ausgangsendes des Atemwegs eingerichtet ist;
wobei der Vorrichtungskörper auch ein Regelsteuersystem enthält, das Regelsteuersystem eine Steuereinheit, eine Ausführungseinheit und eine Erkennungsgseinheit enthält, die Steuereinheit eine Steuerung (45) enthält, die Steuerung in dem CO2-Kompensator (9) für das Atemgas eingerichtet ist, die Steuerung über ein erstes Kabel (71) elektrisch mit dem proximalen Drucksensor (7) verbunden ist, die Steuerung über ein zweites Kabel (81) elektrisch mit dem endtidalen Kohlendioxidsensor (8) verbunden ist, die Ausführungseinheit ein Magnetventil (443) und ein Proportionalventil für die Kohlendioxidregelung (444) enthält, sowohl das Magnetventil als auch das Proportionalventil für die Kohlendioxidregelung auf dem Luftweg (10) eingerichtet sind, das Magnetventil und das Proportionalventil für die Kohlendioxidregelung zwischen dem Einlassfilter (441) und dem Auslassfilter (447) sequenziell eingerichtet sind, das Magnetventil und das Proportionalventil für die Kohlendioxidregelung jeweils elektrisch mit der Steuerung (45) verbunden sind, die Erkennungseinheit einen Einlassdrucksensor (442), einen Kohlendioxid-Durchflussmesser (445) und einen Kohlendioxid-Versorgungsdrucksensor (446) enthält, der sequenziell an den Atemwegen (10) eingerichtet ist, der Einlassdrucksensor (442), der Kohlendioxid-Durchflussmesser (445) und der Kohlendioxid-Versorgungsdrucksensor (446) elektrisch mit der Steuerung (45) verbunden sind, der Einlassdrucksensor (442) sich zwischen dem Einlassfilter (441) und dem Magnetventil (443) befindet, der Kohlendioxid-Durchflussmesser (445) sich zwischen dem Proportionalventil für die Kohlendioxidregelung (444) und dem Auslassfilter (447) befindet, der Drucksensor für die Kohlendioxidversorgung (446) sich zwischen dem Kohlendioxid-Durchflussmesser (445) und dem Auslassfilter (447) befindet;
wobei das Atemparameter-Erkennungsmodul (4) einen Kompensationsmischschlauch (42) enthält, der Kompensationsmischschlauch einen Dreiwegeschlauch verwendet, ein erstes Ende des Kompensationsmischschlauchs (42) für den Luftstrom von dem Beatmungsgerät (2) verwendet wird, ein zweites Ende des Kompensationsmischschlauchs durch einen Verbindungsschlauch (421) mit dem Ausgangsende des Atemwegs (10) verbunden ist, ein Einwegventil (422) in dem zweiten Ende des Kompensationsmischschlauchs (42) eingerichtet ist, ein drittes Ende des Kompensationsmischschlauchs (42) über einen Gasversorgungskreis (11) mit der Innenseite des Atemterminals (5) verbunden ist;
wobei die Erkennungseinheit auch einen Durchflussmesser (41) und einen Kohlendioxidkonzentrationssensor (43) enthält, der Durchflussmesser (41) nahe dem ersten Ende des Kompensationsmischschlauchs (42) eingerichtet ist, der Kohlendioxidkonzentrationssensor (43) nahe dem dritten Ende des Kompensationsmischschlauchs (42) eingerichtet ist, der Durchflussmesser über ein drittes Kabel (411) elektrisch mit der Steuerung (45) verbunden ist, der Kohlendioxidkonzentrationssensor über ein viertes Kabel (412) elektrisch mit der Steuerung (45) verbunden ist.

2. CO2-Kompensationsvorrichtung, die zum Anschluss an einen Universalventilator (2) ausgelegt ist, nach Anspruch 1, wobei
das Atemterminal (5) entweder eine Maske oder eine Beatmungsschlauchintubation ist.

3. CO2-Kompensationsvorrichtung, die zum Anschluss an einen Universalventilator (2) ausgelegt ist, nach Anspruch 1, wobei
eine Außenseite des Luftwegs (10) mit einer Stützplatte (1001) ausgestattet ist, mehrere Schraubenbefestigungslöcher auf der Stützplatte eingerichtet sind, eine Innenseite des Luftwegs (10) mit einer elektrischen Heizplatte (1002) ausgestattet ist, die elektrische Heizplatte zwischen dem Einlassfilter (441) und dem Einlassdrucksensor (442) angeordnet ist, die elektrische Heizplatte elektrisch mit der Steuerung (45) verbunden ist, die elektrische Heizplatte koaxial innerhalb des Luftwegs (10) eingerichtet ist, mehrere Durchgangslöcher (1003) auf der elektrischen Heizplatte (1002) geöffnet sind, eine feste Welle (1004) in der Mitte der elektrischen Heizplatte eingerichtet ist, ein fester Ring (1005) und ein rotierender Ring (1006) nacheinander auf einer Seite der elektrischen Heizplatte (1002) in der Nähe der festen Welle (1004) eingerichtet sind, ein Außenumfang des festen Rings (1005) fest mit einer Innenwand des Atemwegs (10) verbunden ist, ein erster konvexer Ring (1007) auf einer Seite des festen Rings (1005) von der elektrischen Heizplatte (1002) entfernt eingerichtet ist, der erste konvexe Ring koaxial mit dem festen Ring eingestellt ist, ein Außenumfang des rotierenden Rings (1006) drehbar mit der Innenwand des Atemwegs (10) verbunden ist, ein zweiter konvexer Ring (1008) auf einer Seite des rotierenden Rings (1006) in der Nähe des festen Rings (1005) eingerichtet ist, eine Innenseitenwand des zweiten konvexen Rings (1008) drehbar mit einer Außenseitenwand des ersten konvexen Rings (1007) verbunden ist, mehrere Umlenkbleche (1009) auf einem Außenumfang der festen Welle (1004) eingestellt sind, die Umlenkbleche (1009) aus flexiblem Blechmaterial bestehen und in einer kreisförmigen Anordnung um die Mitte der festen Welle angeordnet sind, ein Ende jedes der Umlenkbleche (1009) mit einem äußeren Kreis der festen Welle verbunden ist, ein Ende jedes der Umlenkbleche (1009) von der festen Welle weg mit einer inneren Umfangsfläche des festen Rings auf einer Seite in der Nähe des festen Rings verbunden ist, das Ende jedes der Umlenkbleche (1009) von der festen Welle weg mit einer inneren Umfangsfläche des rotierenden Rings (1006) verbunden ist, eine Antriebskomponente auf einer Seite in der Nähe des rotierenden Rings außerhalb des Atemwegs eingerichtet ist, die Antriebskomponente dazu dient, den rotierenden Ring (1006) zum Drehen anzutreiben.

4. CO2-Kompensationsvorrichtung, die zum Anschluss an einen Universalventilator (2) ausgelegt ist, nach Anspruch 3, wobei
die Antriebskomponente Folgendes enthält: eine Antriebsstange (1010) und eine elektrische Teleskopstange (1011), ein Ende der Antriebsstange durchdringt eine Seitenwand des Atemwegs (10) und ist mit einer Außenwand des rotierenden Rings (1006) verbunden, ein langer Schlitz, der mit der Antriebsstange (1010) kompatibel ist, ist auf dem Atemweg eingerichtet, die Antriebsstange ist gleitend mit dem langen Schlitz verbunden, eine Streifennut (1012) ist an einem Ende der Antriebsstange (1010) von dem rotierenden Ring (1006) entfernt eingerichtet, die elektrische Teleskopstange (1011) ist auf einer Seitenwand der Stützplatte (1001) eingerichtet, ein Ausgangsende der elektrischen Teleskopstange verläuft durch die Streifennut (1012) und ist mit einer Verbindungsstange (1013) ausgestattet, ein Ende der Verbindungsstange (1013) ist scharnierend mit dem Ausgangsende der elektrischen Teleskopstange (1011) verbunden, das andere Ende der Verbindungsstange ist scharnierend mit einer Innenwand eines oberen Endes der Streifennut (1012) verbunden.

5. CO2-Kompensationsvorrichtung, die zum Anschluss an einen Universalventilator (2) ausgelegt ist, nach Anspruch 3, wobei
mehrere Bogenlöcher (1014) an dem rotierenden Ring (1006) geöffnet sind, die Bogenlöcher (1014) in einem kreisförmigen Array um die Mitte des rotierenden Rings (1006) angeordnet sind, eine Führungssäule (1015) in jedem der Bogenlöcher (1014) verschiebbar eingerichtet ist, ein Ende der Führungssäule fest mit einer Seite des festen Rings (1005) in der Nähe des rotierenden Rings (1006) verbunden ist, das andere Ende der Führungssäule sich außerhalb eines der jeweiligen Bogenlöcher (1014) erstreckt und mit einem Begrenzungsblock (1016) ausgestattet ist.

6. CO2-Kompensationssystem, umfassend eine der in den Ansprüchen 1 bis 5 beschriebenen CO2-Kompensationsvorrichtungen, wobei es auch den Ventilator (2) und eine Kohlendioxidgasquelle (1) umfasst, die Kohlendioxidgasquelle (1) über ein Ansaugrohr mit dem Eingangsende des Atemwegs (10) des Atemgas-CO2-Kompensators (9) verbunden ist, der Ventilator (2) über einen Atemkreislauf (13) mit dem Kompensationsmischrohr (42) verbunden ist.

## Revendications

1. Dispositif de compensation de CO2 adapté à être connecté à un ventilateur universel (2), dans lequel
le dispositif inclut : un corps de dispositif, le corps de dispositif comporte un compensateur (9) de CO2 gazeux respiratoire, un module (4) de détection de paramètres respiratoires, et un terminal respiratoire (5), une voie respiratoire (10) est placée à l'intérieur du compensateur (9) de CO2 gazeux respiratoire, une extrémité de sortie de la voie respiratoire (10) est connectée au module (4) de détection des paramètres respiratoires, le module (4) de détection des paramètres respiratoires est connecté au terminal respiratoire (5), un capteur (7) de pression proximal et un capteur (8) de dioxyde de carbone de fin d'expiration sont placés à l'intérieur du terminal respiratoire (5), le capteur (7) de pression proximal est utilisé pour détecter une pression de gaz à l'intérieur du terminal respiratoire (5), le capteur (8) de dioxyde de carbone de fin d'expiration est utilisé pour détecter une valeur de dioxyde de carbone de fin d'expiration à l'intérieur du terminal respiratoire (5), le capteur de pression proximal et le capteur de dioxyde de carbone de fin d'expiration sont connectés électriquement au compensateur (9) de CO2 gazeux respiratoire, respectivement, le compensateur (9) de CO2 gazeux respiratoire délivre du dioxyde de carbone à l'intérieur du terminal respiratoire (5) sur la base de valeurs de détection du capteur (7) de pression proximal et du capteur (8) de dioxyde de carbone de fin d'expiration à travers le module (4) de détection de paramètres respiratoires ;
dans lequel un filtre d'admission (441) est placé près d'une extrémité d'entrée de la voie respiratoire (10), et un filtre (447) d'échappement est placé près de l'extrémité de sortie de la voie respiratoire ;
dans lequel le corps de dispositif inclut également un système de commande de régulation, le système de commande de régulation inclut une unité de commande, une unité d'exécution et une unité de détection, l'unité de commande inclut un dispositif de commande (45), le dispositif de commande est placé à l'intérieur du compensateur (9) de CO2 de gaz respiratoire, le dispositif de commande est connecté électriquement au capteur (7) de pression proximale par l'intermédiaire d'un premier câble (71), le dispositif de commande est connecté électriquement au capteur (8) de dioxyde de carbone de fin d'expiration par l'intermédiaire d'un deuxième câble (81), l'unité d'exécution inclut une électrovanne (443) et une vanne (444) proportionnelle de régulation de dioxyde de carbone, l'électrovanne et la vanne proportionnelle de régulation de dioxyde de carbone sont toutes deux placées sur la voie respiratoire (10), l'électrovanne et la vanne proportionnelle de régulation de dioxyde de carbone sont placées séquentiellement entre le filtre (441) d'admission et le filtre (447) d'échappement, l'électrovanne et la vanne proportionnelle de régulation de dioxyde de carbone sont respectivement connectées électriquement au dispositif de commande (45), l'unité de détection inclut un capteur (442) de pression d'admission, un débitmètre (445) de dioxyde de carbone, et un capteur (446) de pression d'alimentation en dioxyde de carbone placés en séquence sur la voie respiratoire (10), le capteur (442) de pression d'admission, le débitmètre (445) de dioxyde de carbone, et le capteur (446) de pression d'alimentation en dioxyde de carbone sont connectés électriquement au dispositif de commande (45) respectivement, le capteur (442) de pression d'admission est situé entre le filtre (441) d'admission et l'électrovanne (443), le débitmètre (445) de dioxyde de carbone est situé entre la vanne (444) proportionnelle de régulation de dioxyde de carbone et le filtre (447) d'échappement, le capteur (446) de pression d'alimentation en dioxyde de carbone est situé entre le débitmètre (445) de dioxyde de carbone et le filtre (447) d'échappement ;
dans lequel le module (4) de détection de paramètres respiratoires inclut un tube (42) de mélange de compensation, le tube de mélange de compensation utilise un tube à trois voies, une première extrémité du tube (42) de mélange de compensation est utilisée pour le flux d'air provenant du ventilateur (2), une deuxième extrémité du tube de mélange de compensation est connectée à l'extrémité de sortie de la voie respiratoire (10) par l'intermédiaire d'un tube (421) de raccordement, une vanne unidirectionnelle (422) est placée à l'intérieur de la deuxième extrémité du tube (42) de mélange de compensation, une troisième extrémité du tube (42) de mélange de compensation est connectée à l'intérieur du terminal respiratoire (5) par l'intermédiaire d'un circuit (11) d'alimentation en gaz ;
dans lequel l'unité de détection inclut également un débitmètre (41) et un capteur (43) de concentration de dioxyde de carbone, le débitmètre (41) est placé près de la première extrémité du tube (42) de mélange de compensation, le capteur (43) de concentration de dioxyde de carbone est placé près de la troisième extrémité du tube (42) de mélange de compensation, le débitmètre est connecté électriquement au dispositif de commande (45) par le biais d'un troisième câble (411), le capteur de concentration de dioxyde de carbone est connecté électriquement au dispositif de commande (45) par le biais d'un quatrième câble (412).

2. Dispositif de compensation de CO2 adapté à être connecté à un ventilateur universel (2) selon la revendication 1,
dans lequel
le terminal respiratoire (5) est soit un masque, soit une intubation par tube respiratoire.

3. Dispositif de compensation de CO2 adapté à être connecté à un ventilateur universel (2) selon la revendication 1,
dans lequel
un extérieur de la voie respiratoire (10) est équipé d'une plaque de support (1001), plusieurs trous de montage de boulons sont placés sur la plaque de support, un intérieur de la voie respiratoire (10) est équipé d'une plaque chauffante électrique (1002), la plaque chauffante électrique est située entre le filtre (441) d'admission et le capteur (442) de pression d'admission, la plaque chauffante électrique est connectée électriquement au dispositif de commande (45), la plaque chauffante électrique est placée coaxialement à l'intérieur de la voie respiratoire (10), plusieurs trous traversants (1003) sont ouverts sur la plaque chauffante électrique (1002), un arbre fixe (1004) est placé au centre de la plaque chauffante électrique, une bague fixe (1005) et une bague rotative (1006) sont placées séquentiellement sur un côté de la plaque chauffante électrique (1002) près de l'arbre fixe (1004), une circonférence externe de la bague fixe (1005) est reliée fixement à une paroi interne de la voie respiratoire (10), une première bague convexe (1007) est placée sur un côté de la bague fixe (1005) éloigné de la plaque chauffante électrique (1002), la première bague convexe est placée coaxialement avec la bague fixe, une circonférence externe de la bague rotative (1006) est reliée en rotation à la paroi interne de la voie respiratoire (10), une seconde bague convexe (1008) est placée sur un côté de la bague rotative (1006) près de la bague fixe (1005), une paroi latérale interne de la seconde bague convexe (1008) est reliée en rotation à une paroi latérale externe de la première bague convexe (1007), plusieurs déflecteurs (1009) sont placés sur une circonférence externe de l'arbre fixe (1004), les déflecteurs (1009) sont composés d'un matériau en feuille flexible et sont agencés en un réseau circulaire autour du centre de l'arbre fixe, une extrémité de chacun des déflecteurs (1009) est reliée à un cercle externe de l'arbre fixe, une extrémité de chacun des déflecteurs (1009) éloignée de l'arbre fixe est reliée à une surface circonférentielle interne de la bague fixe sur un côté proche de la bague fixe, l'extrémité de chacun des déflecteurs (1009) éloignée de l'arbre fixe est reliée à une surface circonférentielle interne de la bague rotative (1006) sur un côté proche de la bague rotative, un composant d'entraînement est placé à l'extérieur de la voie aérienne, le composant d'entraînement est utilisé pour entraîner la bague rotative (1006) en rotation.

4. Dispositif de compensation de CO2 adapté à être connecté à un ventilateur universel (2) selon la revendication 3,
dans lequel
le composant d'entraînement inclut : une tige d'entraînement (1010) et une tige télescopique électrique (1011), une extrémité de la tige d'entraînement pénètre dans une paroi latérale de la voie respiratoire (10) et est reliée à une paroi externe de la bague rotative (1006), une longue fente compatible avec la tige d'entraînement (1010) est placée sur la voie respiratoire, la tige d'entraînement est reliée de manière coulissante à la longue fente, une rainure en bande (1012) est placée au niveau d'une extrémité de la tige d'entraînement (1010) éloignée de la bague rotative (1006), la tige télescopique électrique (1011) est placée sur une paroi latérale de la plaque de support (1001), une extrémité de sortie de la tige télescopique électrique passe à travers la rainure en bande (1012) et est équipée d'une tige de liaison (1013), une extrémité de la tige de liaison (1013) est reliée de manière articulée à l'extrémité de sortie de la tige télescopique électrique (1011), l'autre extrémité de la tige de liaison est reliée de manière articulée à une paroi interne d'une extrémité supérieure de la rainure en bande (1012).

5. Dispositif de compensation de CO2 adapté à être connecté à un ventilateur universel (2) selon la revendication 3,
dans lequel
plusieurs trous en arc (1014) sont ouverts sur la bague rotative (1006), les trous en arc (1014) sont agencés en un réseau circulaire autour du centre de la bague rotative (1006), une colonne (1015) de guidage est placée de manière coulissante à l'intérieur de chacun des trous en arc (1014), une extrémité de la colonne de guidage est reliée de manière fixe à un côté de la bague fixe (1005) près de la bague rotative (1006), l'autre extrémité de la colonne de guidage s'étend à l'extérieur d'un l'un respectif des trous en arc (1014) et est équipée d'un bloc (1016) de butée.

6. Système de compensation de CO2 comportant l'un quelconque des dispositifs de compensation de CO2 décrits dans les revendications 1 à 5, dans lequel il inclut également le ventilateur (2) et une source (1) de dioxyde de carbone gazeux, la source (1) de dioxyde de carbone gazeux est connectée à l'extrémité d'entrée de la voie respiratoire (10) du compensateur (9) de CO2 gazeux respiratoire par le biais d'un tuyau d'admission, le ventilateur (2) est connecté au tube (42) de mélange de compensation par le biais d'un circuit respiratoire (13).
